# NEUE EUROPÄISCHE PATENTSCHRIFT

(11) **EP 0 505 857 B2**
(45) Veröffentlichungstag und Bekanntmachung des Hinweises auf die Entscheidung über den Einspruch: **24.03.1999**
(45) Hinweis auf die Patenterteilung: 06.12.1995
(21) Anmeldenummer: 92104392.3
(22) Anmeldetag: 13.03.1992
(51) Int. Cl.: A61N 1/39

(54) **Defibrillator/Kardiovertierer**
Defibrillator/cardioverter
Défibrillateur cardiaque/cardiovèrtre

(30) Priorität: 28.03.1991 DE 4110402
(43) Veröffentlichungstag der Anmeldung: 30.09.1992
(73) Patentinhaber: Pacesetter AB, 175 84 Järfälla (SE)
(72) Erfinder: Hirschberg, Jakub, S-183 44 Täby (SE); Obel, Martin, S-182 33 Danderryd (SE)
(74) Vertreter: Harrison, Michael Charles

(56) Entgegenhaltungen:
- EP-A- 0 102 201
- EP-B- 0 023 134
- DE-A- 3 503 854
- DE-A- 3 919 498
- US-A- 4 548 203
- US-A- 4 821 723
- US-A- 4 834 100

## Beschreibung

Die Erfindung betrifft einen Defibrillator/Kardiovertierer mit einer Mehrzahl von n (n ≧ 3) Elektroden die an einer Einrichtung zur Erzeugung elektrischer Impulse angeschlossen sind.

Bei einem derartigen, aus der DE-A-39 19 498 bekannten Defibrillator oder Kardiovertierer ist eine von mehreren Elektroden im Herzinneren angeordnet und die übrigen Elektroden außen am Herzen plaziert. Die äußeren Elektroden sind entweder unmittelbar oder über für hohe Spannungen jeweils einen Kurzschluß bildende Entladungsstrecken elektrisch miteinander verbunden und an einem ersten von zwei Ausgangsanschlüssen des Ausganges eines Impulsgenerators angeschlossen; die im Herzinneren angeordnete Elektrode ist mit dem zweiten Ausgangsanschluß verbunden. Damit wird erreicht, daß sich bei der Abgabe eines elektrischen Impulses durch den Impulsgenerator die elektrische Stromdichte im Herzmuskel entsprechend der Anordnung der Elektroden verteilt und dabei vorzugsweise die dicksten Zonen des Herzmuskels, die den Haupteil der Herzmuskelmasse bilden, durchdringt, um eine Defibrillation bzw. Kardioversion zu erzielen.

Aus der US-A-4 548 203 ist ein weiterer Defibrillator mit mehreren Elektroden bekannt, die paarweise an unterschiedliche Ausgänge eines Impulsgenerators angeschlossen sind und an unterschiedlichen, vorzugsweise einander gegenüberliegenden Stellen des Herzens plaziert sind. Zur Defibrillation des Herzens werden die einzelnen Elektrodenpaare nacheinander über die Ausgänge des Impulsgenerators mit jeweils einem elektrischen Impuls beaufschlagt. Durch die sowohl räumlich als auch zeitlich getrennte Impulsabgabe soll eine Herabsetzung der zur Auslösung der Defibrillation erforderlichen Energie erreicht werden.

Bei dem aus der DE-A-39 19 498 bekannten Defibrillator läßt sich die Stromverteilung im Herzen nur durch die Anordnung und Größe der einzelnen Elektroden einstellen. Die Anordnung der Elektroden und insbesondere ihr Abstand zueinander und zum Herzen ist jedoch durch die anatomischen Gegebenheiten beschränkt. Außerdem ist eine im Herzinneren angeordnete Elektrode erforderlich, in deren näherer Umgebung eine sehr hohe Stromdichte entsteht, durch die bei einer ungünstigen Plazierung oder einer Verlagerung der inneren Elektrode eine Beschädigung des Herzgewebes nicht ausgeschlossen werden kann.

Da bei dem aus der US-A-4 548 203 bekannten Defibrillator jeweils nur zwei Elektroden gleichzeitig bei der Impulsabgabe beteiligt sind, ist eine Verteilung der Stromdichte auf unterschiedliche Zonen des Herzmuskels kaum möglich.

Der Erfindung liegt die Aufgabe zugrunde, einen Defibrillator bzw. Konverter anzugeben, mit dem sich eine optimale Stromverteilung im Herzen zur Erzielung einer möglichst geringen Defibrillationsschwelle einstellen läßt.

Gemäß der Erfindung wird die Aufgabe dadurch gelöst daß bei dem Defibrillator/Konverter der eingangs angegebenen Art die Einrichtung zur Erzeugung elektrischer Impulse n -1 in Reihe geschaltete Ausgänge aufweist, an deren insgesamt Ausgangsanschlüssen die Elektroden angeschlossen sind, und daß die Einrichtung an jedem ihrer Ausgänge jeweils gleichzeitig einen elektrischen Impuls abgibt. Dadurch wird erreicht, daß die Stromverteilung im Herzen nicht nur von der Anordnung der Elektroden abhängig ist, sondern zusätzlich über die elektrischen Spannungen an den Ausgängen der Einrichtung zur Erzeugung der elektrischen Impulse eingestellt werden kann. Es besteht daher auch nicht die Gefahr, daß es aufgrund der Anordnung der Elektroden zur einer Gewebeschädigung bei der Abgabe des elektrischen Impulses kommen kann, weil der Impulsstrom für jede einzelne Elektrode einstellbar ist.

Entsprechend einer vorteilhaften Ausfgührung des erfindungsgemäßen Defibrillators weist die Einrichtung zur Erzeugung elektrischer Impulse n -1 in Reihe geschaltete Kondensatoren auf, die zum Aufladen über eine Schalteranordnung an eine Spannungsquelle schaltbar sind; die Anschlüsse der einzelnen Kondensatoren sind über steuerbare Schalter mit den Ausgangsanschlüssen der Einrichtung verbunden. Solange die Schalteranordnung geschlossen ist, wird die Reihenschaltung aus den Kondensatoren auf die von der Spannungsquelle vorgegebene Ladespannung aufgeladen, wobei das Verhältnis der Teilspannungen über den einzelnen Kondensatoren und damit an den Ausgängen der Einrichtung zur Erzeugung elektrischer Impulse von den Kapazitätswerten der einzelnen Kondensatoren abhängig und damit einstellbar ist.

Um im Falle eines Kurzschlusses eine Strombegrenzung zu erreichen, sind vorteilhafter Weise in den Leitungswegen zwischen den Anschlüssen der Kondensatoren und den Ausgangsanschlüssen der Einrichtung Induktivitäten eingefügt. Ein weiterer Vorteil besteht darin, daß bei der Entladung der Kondensatoren über die Induktivitäten und den elektrischen Widerstand des Herzgewebes zwischen den einzelnen Elektroden biphasische Stromimpulse in Form von stark gedämpften Schwingungen erzeugt werden; eine derartige Impulsform hat sich in Bezug auf die zur Defibrillation benötigte Energie als besonders effektiv erwiesen.

Bei einer zu der obengenannten Reihenschaltung von Kondensatoren alternativen Ausführungsform des erfindungsgemäßen Defibrillators/Konverters weist die Einrichtung zur Erzeugung elektrischer Impulse einen Impulsgenerator mit zwei Ausgangspolen auf zwischen denen und den n Ausgangsanschlüssen eine Stromteilerschaltung bestehend aus passiven elektrischen Bauelementen liegt Die Stromteilerschaltung teilt den von dem Impulsgeneralor kommenden Defibrillationsimpuls in unterschiedliche Teilimpulse für die einzelnen Elektroden auf wobei die Form und Amplitude der einzelnen Teilimpulse durch die Auswahl und Anordnung der passiven elektrischen Bauelemente (Induktivitäten. Widerstände. Kondensatoren) bestimmt ist.

Entsprechend einer besoders vorteilhaften Weiterbildung des erfindungsgemäßen Defibrillators/Konverters ist die Einrichtung zur Erzeugung elektrischer Impulse in einem implantierberen Kapselgehäuse angeordnet, das eine zumindest bereichsweise elektrisch leitende und als eine der Elektroden ausgebildete Oberfläche aufweist. Das Kapselgehäuse wird dann in der Nähe des Herzens implantiert und übernimmt dort zusätzlich die Funktion einer Elektrode.

Im Zusammenhang mit der obengenannten, eine Stromteilerschaltung enthaltenden Ausbildung des erfindungsgemäßen Defibrillators/Konverters ist entsprechend einer vorteilhaften Weiterbildung vorgesehen, daß der Impulsgenerator einerseits und die Stromteilerschaltung andererseits in separaten implantierbaren Gehäusen untergebracht und über elektrische Zuleitungen miteinander verbindbar sind. Dadurch ist es insbesondere möglich, bereits vorhandene herkömmliche Defibrillatoren, bei denen ein Impulsgenerator einen Defibrillationsimpuls zwischen zwei Ausgangspolen erzeugt, im Sinne des erfindungsgemäßen Defibrillators zur Abgabe unterschiedlicher Stromimpulse an verschiedene Elektroden zu erweitern. Da das die Stromteilerschaltung beinhaltende Gehäuse im Vergleich zu dem Gehäuse mit dem Impulsgenerator geringere Abmessungen aufweisen kann, läßt es sich in einer relativ unaufwendigen Operation zusätzlich zu dem bereits vorhandenen Impulsgenerator in dem Körper des Patienten implantieren.

Indem das die Stromteilerschaltung und/oder das den Impulsgenerator beinhaltende Gehäuse eine zumindest bereichsweise elektrisch leitende und als eine der Elektroden ausgebildete Oberfläche aufweist, können beide Gehäuse zusätzlich die Funktion einer der Elektroden übernehmen, wodurch der Aufwand beim Implantieren vermindert wird.

Zur Erläuterung der Erfindung wird im folgenden auf die Figuren der Zeichnung Bezug genommen; im einzelnen zeigen
FIG 1 den Schaltungsaufbau eines ersten Ausführungsbeispiels des ertindungsgemäßen Defibrillators.
FIG 2 den in FIG 1 gezeigten Defibrillator in einem implantierbaren und die Funktion einer Elektrode übernehmenden Gehäuse.
FIG 3 die Anordnung des in FIG 2 gezeigten Defibrillators zusammen mit zwei weiteren Elektroden im menschlichen Körper.
Fig 4 ein Defibrillator mit einem Impulsgenerator und einer Stromteilerschaltung und
FIG 5 die Anordnung des Impulsgenerators und der Stromteilerschaltung in separaten implantierbaren Gehäusen.

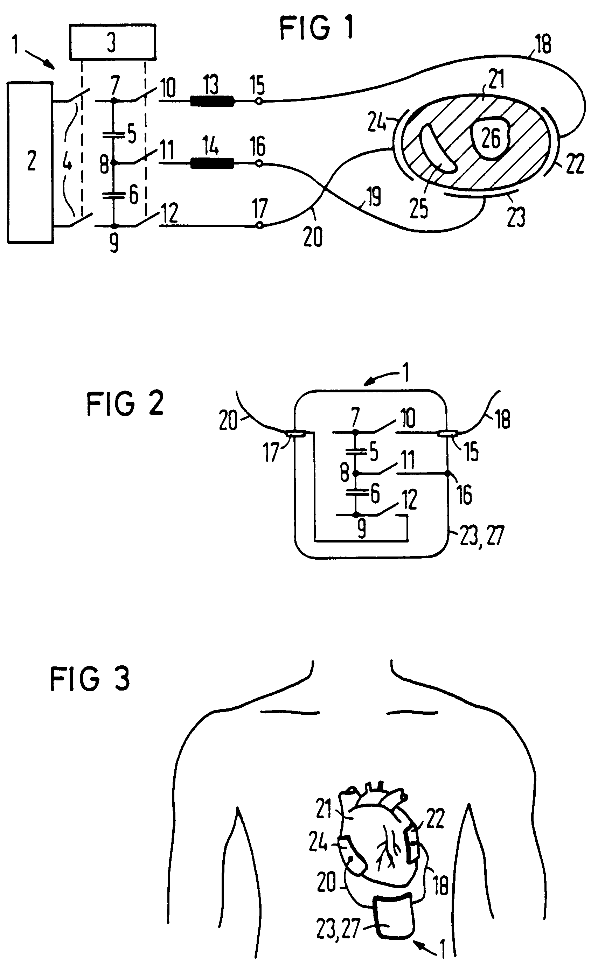

In Fig 1 ist mit 1 eine Einrichtung zur Erzeugung elektrischer Impulse bezeichnet. Die Einrichtung 1 enthält eine Spannungsquelle 2, die über eine von einer Steuereinrichtung 3 steuerbare Schalteranordnung 4 an zwei in Reihe liegende Kondensatoren 5 und 6 schaltbar ist. Die Reihenschaltung der Kondensatoren 5 und 6 weist drei unterschiedliche Anschlußstellen 7,8 und 9 auf, die jeweils über steuerbare Schalter 10,11 und 12 sowie im Falle der mit 7 und 8 bezeichneten Anschlußstellen jeweils über Induktivitäten 13 und 14 mit Ausgangsanschlüssen 15,16 und 17 der Einrichtung 1 verbunden sind. Die steuerbaren Schalter 10, 11 und 12 sind jeweils gleichzeitig durch die Steuereinrichtung 3 ansteuerbar. Entspechend der Anzahl der in Reihe liegenden Kondensatoren 5 und 6 bilden die Ausgangsanschlüsse 15,16 und 17 zwei in Reihe liegende Ausgänge der impulserzeugenden Einrichtung 1, die über Elektrodenleitungen 18,19 und 20 mit an einem hier im Querschnitt dargestellten Herzen 21 angeordneten Elektroden 22,23 und 24 verbunden sind. Bei dem gezeigten Ausführungsbeispiel kann die Anzahl der Elektroden ohne weiteres auf n (n > 3) erweitert werden. wobei n-1 in Reihe liegende Kondensatoren an n-1 Ausgänge mit n Ausgangsanschlüssen für die n Elektroden schaltbar sind.

Solange die Schalteranordnung 4 geschlossen ist, wird die Reihenschaltung aus den Kondensatoren 5 und 6 auf die von der Spannungsquelle 2 vorgegebene Ladespannung aufgeladen, wobei das Verhältnis der Teilspannungen über den einzelnen Kondensatoren 5 und 6 von deren Kapazität abhängig ist. Zur Defibrillation des Herzens 24 werden die Schalter 10,11 und 12 geichzeitig geschlossen. so daß sich die Kondensatoren 5 und 6 über die Induktivitäten 13 und 14 sowie das zwischen den Elektroden 22,23 und 24 liegende Herzgewebe entladen. Dem Herzen 21 werden also über die Elektroden 22,23 und 24 jeweils gleichzeitig Stromimpulse zugeführt. deren jeweilige Impulshöhe bzw. Energiemenge von den Teilspannungen über den Kondensatoren 5 und 6, den Indutivitäten 13 und 14 sowie den Teilimpedanzen des Herzgewebes zwischen den Elektroden 22 und 23, 23 und 24 sowie 22 und 24 abhängig ist. Durch die Induktivitäten 13 und 14 erhalten die Stromimpulse die Form von biphasischen, stark gedämpften Schwingungen, die sich als besonders günstig zur Erzielung einer effektiven Defibrillation erwiesen haben Die Induktivitäten 13 und 14 dienen ferner zur Strombegrenzung im Falle eines Kurzschlußlehlers in der Einrichtung 1.

Wie FIG 1 zeigt, ist die Herzmuskelmasse über den Querschnitt des Herzens 21 ungleichmäßig verteilt. Wenn man für die Dicke des Herzmuskels zwischen dem rechten Ventrikel 25 und der benachbarten Herzaußenwand den normierten Wert 1 ansetzt, ergibt sich näherungsweise für die Herzmuskelmasse zwischen dem rechten Ventrikel 25 und dem linken Ventrikel 26 der Wert 2, für die Herzmuskelmasse zwischen dem linken Ventrikel 26 und der Herzaußenwand im Bereich der Elektrode zu 22 der Wert 3 und für die Herzmuskelmasse zwischen dem linken Ventrikel 26 und der Herzaußenwand im Bereich der Elektrode 23 der Wert 2. Indem die Kapazitätsverhältnisse der Kondensatoren 5 und 6 so gewählt wurden, daß die Teilspannung an dem Kondensator 5 in der Größenordnung von 2/3 und die Teilspannung an dem Kondensator 6 in der Größenordnung von 1/3 der gesamten Spannung zwischen den Anschlußstellen 7 und 9 beträgt, wurde eine alle Bereiche des Herzmuskels gleichmäßig erfassende Stromverteilung erzielt und die zur Defibrillierung erforderliche Energie auf etwa 60 bis 80 Prozent der bei einer Defibrillation mit nur zwei Elektroden erforderlichen Energiemenge reduziert.

Bei dem in FIG 1 gezeigten Ausführungsbeispiel kann es sich um einen implantierbaren oder nichtimplantierbaren Defibrillator handeln. Dementsprechend können die Elektroden 22,23 und 24 direkt am Herzen 21 oder außen am Körper des Patienten in entsprechender Ausrichtung zum Herzen 21 angeordnet werden.

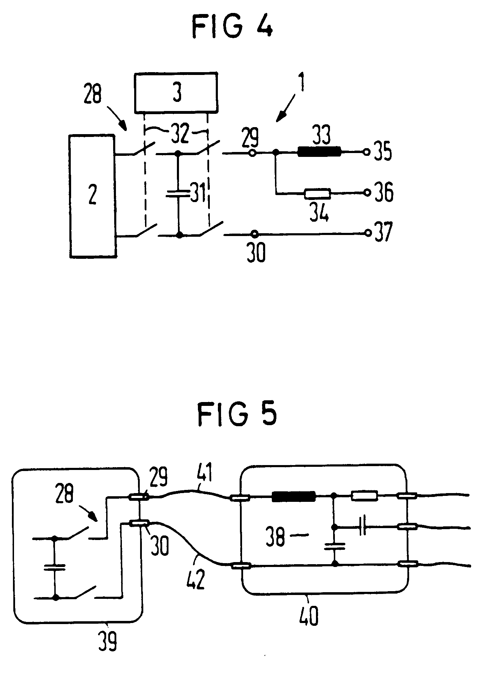

FIG 2 zeigt ein Ausführungsbeispiel für eine Anordnung der impulserzeugenden Einrichtung 1 in einem implantierbaren Kapselgehäuse 27, das vorzugsweise aus Titan besteht. Zur Vereinfachung der Darstellungsweise sind im Inneren des Kapselgehäuses 27 lediglich die beiden Kondensatoren 5 und 6 mit dem steuerbaren Schaltern 10,11 und 12 gezeigt. Die mit den Anschlußstellen 7 und 9 verbundenen Ausgangsanschlüsse 15 und 17 sind als Isolationsdurchführungen durch das Metallgehäuse 27 ausgebildet und, wie FIG 3 zeigt, über die Elektrodenleitungen 18 und 20 mit den direkt am Herzen 21 applizierten Elektroden 22 und 24 verbunden. Die Anschlußstelle 8 zwischen den Kondensatoren 5 und 6 ist über den Schalter 11 direkt mit dem Metallgehäuse 27 verbunden, das die Funktion der Elektroden 23 übernimmt.

FIG 4 gehört nicht zur Offenbarung der Erfindung und dient lediglich zur Verständigung der FIG 5.

FIG 4 zeigt ein Defibrillator, bei dem die Einrichtung 1 zur Erzeugung elektrischer Impulse einen Impulsgenerator 28 mit zwei Ausgangspolen 29 und 30 aufweist. Der Impulsgenerator 28 ist wie ein herkömmlicher Defibrillator aufgebaut und enthält einen Kondensator 31, der mittels einer von einer Steuereinrichtung 3 gesteuerten Schalteranordnung 32 entweder zum Aufladen an eine Spannungsquelle 2 oder zur Abgabe eines Defibrillalionsimpulses an die beiden Ausgangspole 29 und 30 des Impulsgenerators 28 schaltbar ist. Die beiden Ausgangspole 29 und 30 sind über eine aus passiven elektrischen Bauelementen, hier einer Induktivität 33 und einem Widerstand 34, bestehende Stromteilerschaltung mit drei Ausgangsanschlüssen 35,36 und 37 der impulserzeugenden Einrichtung 1 verbunden. An den Ausgangsanschlüssen 35,36 und 37 sind entsprechend der Darstellung in FIG 1 hier nicht gezeigte Elektrodenleitungen mit Elektroden anschließbar.

FIG 5 zeigt schließlich, daß der Impulsgenerator 28 und die Stromteilerschaltung 38 in zwei separaten implantierbaren Gehäusen 39 und 40 untergebracht sein können, die über elektrische Zuleitungen 41 und 42 miteinander verbunden sind. Dadurch ist es insbesondere ermöglicht, bereits vorhandene herkömmliche, dem Impulsgenerator 28 entsprechende Defibrillatoren im Sinne des erfindungsgemäßen Defibrillators zur Abgabe unterschiedlicher Stromimpulse an mehrere Elektroden zu erweitern. Da das separate Gehäuse 40 im Vergleich zu dem Gehäuse 39 geringere Abmessungen aufweisen kann, läßt es sich in einer relativ unaufwendigen Operation zusätzlich zu dem bereits vorhandenen Impulsgenerator 28 in dem Körper des Patienten implantieren.

Entsprechend dem in FIG 2 gezeigten Ausführungsbeispiel kann auch das die Stromteilerschaltung 38 beinhaltende Gehäuse 40 als Elektrode ausgebildet werden. Außerdem ist die in den

Figuren 4 und 5 gezeigte Auswahl und Anordnung von passiven Bauelementen lediglich als Beispiel für eine Vielzahl weiterer möglicher Ausführungsformen zu verstehen.

### Bezugszeichenliste

- 1: Einrichtung zur Erzeugung elektrischer Impulse
- 2: Spannungsquelle
- 3: Steuereinrichtung
- 4: Schalteranordnung
- 5,6: Kondensatoren
- 7,8,9: Anschlüsse der Kondensatoren 5 und 6
- 10,11,12: steuerbare Schalter
- 13,14: Induktivitäten
- 15,16,17: Ausgangsanschlüsse von 1
- 18,19,20: Elektrodenleitungen
- 21: Herz
- 22,23,24: Elektroden
- 25: rechter Ventrikel
- 26: linker Ventrikel
- 27: Kapselgehäuse
- 28: Impulsgenerator
- 29,30: Ausgangspole von 28
- 31: Kondensator
- 32: steuerbare Schalteranordnung
- 33: Induktivität
- 34: Widerstand
- 35,36,37: Ausgangsanschlüsse von 1
- 38: Stromteilerschaltung
- 39: implantierbares Gehäuse für 28
- 40: separates Gehäuse für 38
- 41,42: elektrische Zuleitungen zwischen 39 und 40

## Patentansprüche

1. Defibrillator/Konverter mit einer Mehrzahl von n mit n ≧ 3 Elektroden (22,23,24), die an einer Einrichtung (1) zur Erzeugung elektrischer Impulse angeschlossen sind, **dadurch gekennzeichnet,** daß die Einrichtung (1) zur Erzeugung elektrischer Impulse n-1 in Reihe geschaltete Ausgänge aufweist, an deren insgesamt n Ausgangsanschlüssen (15,16,17) die Elektroden (22,23,24) angeschlossen sind, und daß die Einrichtung (1) an jedem ihrer Ausgänge jeweils gleichzeitig einen elektrischen Impuls abgibt.

2. Defibrillator/Konverter nach Anspruch 1, **dadurch gekennzeichnet,** daß die Einrichtung (1) zur Erzeugung elektrischer Impulse n-1 in Reihe geschaltete Kondensatoren (5,6) aufweist, die zum Aufladen über eine Schalteranordnung (4) an eine Spannungsquelle (2) schaltbar sind, und daß die Anschlüsse (7,8,9) der einzelnen Kondensatoren (5,6) über steuerbare Schalter (10,11,12) mit den Ausgangsanschlüssen (15,16,17) der Einrichtung (1) verbunden sind.

3. Defibrillator/Konverter nach Anspruch 2, **dadurch gekennzeichnet,** daß in den Leitungswegen zwischen den Anschlüssen (7,8,9) der Kondensatoren (5,6) und den Ausgangsanschlüssen (15,16,17) der Einrichtung (1) Induktivitäten (13,14) eingefügt sind.

4. Defibrillator/Konverter nach Anspruch 1, **dadurch gekennzeichnet,** daß die Einrichtung (1) zur Erzeugung elektrischer Impulse einen Impulsgenerator (28) mit zwei Ausgangspolen (29,30) aufweist, zwischen denen und den n Ausgangsanschlüssen (35,36,37) eine stromteilerschaltung bestehend aus passiven elektrischen Bauelementen (33,34) liegt.

5. Defibrillator/Konverter nach einem der vorangehenden Ansprüche, **dadurch gekennzeichnet,** daß die Einrichtung (1) zur Erzeugung elektrischer Impulse in einem implantierbaren Kapselgehäuse (27) angeordnet ist und daß das Kapselgehäuse (27) eine zumindest bereichsweise elektrisch leitende und als eine der Elektroden (23) ausgebildete Oberfläche aufweist.

6. Defibrillator/Konverter nach Anspruch 4, **dadurch gekennzeichnet,** daß der Impulsgenerator (28) einerseits und die Stromteilerschaltung (38) andererseits in separaten implantierbaren Gehäusen (39,49) untergebracht und über elektrische Zuleitungen (41,42) miteinander verbindbar sind.

7. Defibrillator/Konverter nach Anspruch 6, **dadurch gekennzeichnet,** daß das die Stromteilerschaltung beinhaltende Gehäuse eine zumindest bereichsweise elektrisch leitende und als eine der Elektroden ausgebildete Oberfläche aufweist.

8. Defibrillator/Konverter nach Anspruch 6 oder 7, **dadurch gekennzeichnet,** daß das den Impulsgenerator beinhaltende Kapselgehäuse eine zumindest bereichsweise elektrisch leitende und als eine der Elektroden ausgebildete Oberfläche aufweist.

## Claims

1. Defibrillator/converter having a plurality of n, with n ≧ 3, electrodes (22, 23, 24), which are connected to a device (1) for generating electric pulses, characterised in that the device (1) for generating electric pulses has n-1 outputs which are connected in series and have a total of n output terminals (15, 16, 17) to which the electrodes (22, 23, 24) are connected, and in that the device (1) emits an electric pulse at each of its outputs, simultaneously in each case.

2. Defibrillator/converter according to claim 1, characterised in that the device (1) for generating electric pulses has n-1 capacitors (5, 6) which are connected in series and can be connected by way of a switching arrangement (4) to a voltage source (2) for charging, and in that the terminals (7, 8, 9) of the individual capacitors (5, 6) are connected by way of controllable switches (10, 11, 12) to the output terminals (15, 16, 17) of the device (1).

3. Defibrillator/converter according to claim 2, characterised in that inductors (13, 14) are inserted into the line paths between the terminals (7, 8, 9) of the capacitors (5, 6) and the output terminals (15, 16, 17) of the device (1).

4. Defibrillator/converter according to claim 1, characterised in that the device (1) for generating electric pulses has a pulse generator (28) having two output poles (29, 30), between which poles and the n output terminals (35, 36, 37) there is a current dividing circuit arrangement consisting of passive electric components (33, 34).

5. Defibrillator/converter according to one of the preceding claims, characterised in that the device (1) for generating electric pulses is arranged in an implantable capsule housing (27) and in that the capsule housing (27) has a surface which is electroconductive at least in areas and is formed as one of the electrodes (23).

6. Defibrillator/converter according to claim 4, characterised in that the pulse generator (28) on the one hand and the current dividing circuit arrangement (38) on the other hand are located in separate implantable housings (39, 49(sic)) and can be connected to each other by way of electric supply lines (41, 42).

7. Defibrillator/converter according to claim 6, characterised in that the housing which contains the current dividing circuit arrangement has a surface which is electroconductive at least in areas and is formed as one of the electrodes.

8. Defibrillator/converter according to claim 6 or 7, characterised in that the capsule housing which contains the pulse generator has a surface which is electroconductive at least in areas and is formed as one of the electrodes.

## Revendications

1. Défibrillateur/convertisseur comportant une multiplicité de n électrodes (22,23,24), qui sont raccordées à un dispositif (1) pour produire des impulsions électriques, avec n ≧ 3, caractérisé par le fait que le dispositif (1) servant à produire des impulsions électriques comporte n-1 sorties branchées en série, que les électrodes (22,23,24) sont raccordées à un ensemble de n bornes de sortie (15,16,17) de ces sorties et que le dispositif (1) délivre respectivement et simultanément une impulsion électrique à chacune de ses sorties.

2. Défibrillateur/convertisseur suivant la revendication 1, caractérisé par le fait que le dispositif (1) servant à produire des impulsions électriques comporte n-1 condensateurs (5,6) branchés en série, qui peuvent être commutés pour se charger à une source de tension (2) par l'intermédiaire d'un dispositif à interrupteurs (4), et que les bornes (7,8,9) des différents condensateurs (5,6) sont reliées, par l'intermédiaire d'interrupteurs commandables (10,11,12), aux bornes de sortie (15,16,17) du dispositif (1).

3. Défibrillateur/convertisseur suivant la revendication 2, caractérisé par le fait que les inductances (13,14) sont insérées dans les parties de lignes entre les bornes (7,8,9) des condensateurs (5,6) et les bornes de sortie (15,16,17) du dispositif (1).

4. Défibrillateur/convertisseur suivant la revendication 1, caractérisé par le fait que le dispositif (1) servant à produire des impulsions électriques possède un générateur d'impulsions (28) comportant deux bornes de sortie (29,30), et qu'un circuit diviseur de courant constitué par des composants électriques passifs (33,34) est disposé entre ces bornes de sortie et les n bornes de sortie (35,36,37).

5. Défibrillateur/convertisseur suivant l'une des revendications précédentes, caractérisé par le fait que le défibrillateur (1) servant à produire des impulsions électriques est disposé dans un boîtier d'encapsulation implantable (27) et que le boîtier d'encapsulation (27) possède une surface électriquement conductrice au moins par zones et est agencé de manière à constituer l'une des électrodes (23).

6. Défibrillateur/convertisseur suivant la revendication 4, caractérisé par le fait que le générateur d'impulsions (28), d'une part, et le circuit diviseur de courant (38), d'autre part, sont logés dans des boîtiers implantables séparés (39,49) et peuvent être reliés entre eux par l'intermédiaire de lignes d'alimentation électrique (41, 42).

7. Défibrillateur/convertisseur suivant la revendication 6, caractérisé par le fait que le boîtier contenant le circuit diviseur de courant possède une surface électriquement conductrice, au moins par zones, et agencée de manière à constituer l'une des électrodes.

8. Défibrillateur/convertisseur suivant la revendication 6 ou 7, caractérisé par le fait que le boîtier d'encapsulation qui contient le générateur d'impulsions, possède une surface électriquement conductrice, au moins par zones, et agencée pour constituer l'une des électrodes.
